# EUROPEAN PATENT APPLICATION

(11) **EP 3 301 121 A1**
(43) Date of publication of application: **04.04.2018**
(21) Application number: 16191842.0
(22) Date of filing: 30.09.2016
(51) Int. Cl.: C08G 65/26, C08G 65/331, C08G 65/332, C08G 65/22

(54) **A THERMORESPONSIVE POLYMER AND ITS USE IN A CELL CULTURE SUPPORT**

(71) Applicant: Freie Universität Berlin, 14195 Berlin (DE)
(72) Inventor: DR. WEINHART, Heidemarie, 12435 Berlin (DE); STÖBENER, Daniel, 12163 Berlin (DE)
(74) Representative: Maikowski & Ninnemann Patentanwälte Partnerschaft mbB

(57) **Abstract**

The present invention relates to a thermoresponsive polymer comprising a linear polyether chain of the general formulae (I) Nu-[CH₂-CR¹R²O]ₘ-[CH₂-CR³R⁴O]ₘ-[CH₂-CR⁵R⁶O]ₒ-[CH₂-CR⁷R⁸O]ₚ-H; wherein R¹, R³, R⁵ and R⁷ are in each case H or a C₁-C₁₀ alkyl side chain, wherein R¹, R³, R⁵ and R⁷ may be the same or different; R² and R⁴ are in each case a side chain comprising at least one thermoresponsive moiety, wherein R² and R⁴ may be the same or different; R⁶ and R⁸ are in each case H, a moiety containing at least one C=C double bond or a photo-crosslinkable moiety selected from a group of aromatic and heteroaromatic compounds, wherein R⁶ and R⁸ may the same or different, wherein at least one of R⁶ and R⁸ has to be a photo-crosslinkable moiety, Nu is nucleophilic moiety selected from a group comprising halogen or alkoxy; m, n are in each case or in sum 10-5000, preferably 10-1000, more preferably 100-500; o, p are in each case 1-20 or in sum 2-20, preferably 3-15, more preferably 5-10. The invention also relates to a cell substrate support comprising the polymer.

## Description

The present invention relates to a thermoresponsive polymer according to claim 1, a method for obtaining the same according to claim 7 and a cell culture support comprising the same according to claim 11.

### Description

Thermoresponsive polymers (TRP) are synthetic polymers that can undergo responsive behavior to external influences such as temperature. The polymers respond to a change in temperature by conformational changes. This temperature dependence can be finely tuned and makes thermoresponsive polymers attractive as coatings for cell culture applications.

Thermoresponsive polymers allow adhesion and proliferation of cells and thus the culturing of cell sheets. Due to their responsive behavior, it is possible to harvest confluent cell sheets from polymer coated substrates or cell culture supports by a simple change in temperature. The temperature change (typically by reducing the temperature from 37 °C to room temperature) causes a spontaneous detachment of the cell sheet. Mechanistically this is currently not well understood but often the effect is attributed to a change of the polymer chains from a hydrophobic to a more hydrophilic nature at their switching / volume phase transition temperature.

The temperature change is advantageously employed to safely harvest cell sheets grown on the polymeric layer of the cell culture support. In the hydrophobic state of the TRPs, the cell supports generally become more favorable for cells to attach and grow on the polymer coating. As compared to the use of proteolytic enzymes (e.g. trypsin) or mechanical scraping to harvest cell sheets, the use of TRPs to harvest confluent cell sheets minimizes damage to cells and their excreted extracellular matrix (ECM), thus preserving their biological functions.

It has been discussed in the past that the thickness or density of the polymer coating is an essential factor to successfully culture cells and detach confluent cell sheets from the culture substrate.

For example, it was found for poly(N-isopropyl acryl amide) (pNIPAm) that a polymer layer thickness of 20-34 nm resulted in sufficient cell adhesion and proliferation as well as temperature induced detachment. In contrast, with increasing polymer layer thickness (200 nm) decreasing protein adsorption was detected on the polymer coating which hampered cell attachment and thus impedes successful cell culture (Nash et al., Journal of Materials Chemistry, 2012, 22, 19376).

In order to coat polymers on substrates with control of the resulting layer thickness different approaches are commonly used. Generally, these can be classified into grafting-from and grafting-to processes. In the grafting-from approach monomers are polymerized directly from the cell culture substrate. Here, radical polymerizations like the electron beam polymerization (EBP), the plasma polymerization and the UV-initiated polymerization are the most commonly used methods.

The EBP is a method in which a monomer like NIPAm is homogeneously spread over the cell culture substrate and exposed to an electron beam, which induces polymerization and leads to the covalent attachment of pNIPAm polymer chains to the substrate. It was shown that when the thickness of the coating exceeded 30 nm cells can no longer attach to it and that the optimum thickness of the coating is between 15 and 20 nm (Akiyama et al., Langmuir, 2004, 20, 5506; Fukumori et al., Macromol. Biosci., 2010, 10, 1117).

In an alternative approach, the NIPAm monomer is deposited onto the cell culture substrate from the vapor phase in a plasma glow discharge polymerization. The high energies and temperatures employed in this process can lead to the decomposition of the monomer as well as the formed polymer chains and can lead to their loss of chemical functionality, which can in turn lead to a loss in response of the grafted layer to temperature in the application as cell culture substrate (Canavan et al., J. Biomed. Mater. Res. Part A, 2005, 75, 1; Pan et al., Biomacromolecules, 2001, 2, 32). However, it has been shown that cell growth is independent on the grafted pNIPAm layer thickness (Canavan et al., Langmuir, 2005, 21, 1949; Kumashiro et al., Ann. Biomed. Eng., 2010, 38, 1977).

The main drawbacks of the EB- and plasma polymerization are that they are cost-intensive, require complicated and expensive machinery, and may be dependent on the substrate material and geometry and that they suffer from batch to batch inconsistencies.

A milder approach is the photo-polymerization with UV light which was first utilized by Morra et al and in which the monomer is grafted from the substrate from organic or aqueous solutions containing a photoinitiator, for example benzophenone (Morra et al., Surface Modification of Polymeric Biomaterials, 1997, 175).

An alternative to the above mentioned free radical grafting-from approaches are controlled living radical polymerization (CLRP) methods. These enable the functionalization of cell culture substrates with well-defined polymer architectures, for example thermoresponsive polymer brushes. For example, Linhui et al. grafted pNIPAm onto silicon wafers via surface-initiated atom transfer radical polymerization (SI-ATRP) and found that brush thicknesses of 20-45 nm allowed the satisfactory cell attachment and detachment, while thicker coatings (80-200 nm) showed to be too hydrophilic for cells to attach to the substrate, whereas cells barely detached on brush layers thinner than 20 nm (Linhui et al. Langmuir, 2008, 24, 13632). Mizutani et al. observed a similar trend when they functionalized polystyrene substrates with pNIPAm brushes via SI-ATRP (Mizutani et al., Biomaterials, 2008, 29, 2073).

Wischerhoff et al. used a different class of thermoresponsive polymers to coat gold and glass substrates via SI-ATRP. They copolymerized 2-(2-methoxyethoxy)ethyl methacrylate (MEO₂MA) and oligo(ethylene glycol) methacrylate (OEGMA) and obtained 65 nm thick surface coatings with which they were able to control cell spreading. However, the cell adhesion was generally poor and cells did not proliferate to confluent cell sheets without performing multiple culture cycles (Wischerhoff et al., Angew. Chem. Int. Ed., 2008, 47, 5666; Sefcik et al., Cell. Mol. Bioeng., 2013, 6(3), 287).

Similarly, Dworak et al. grew poly[tri(ethylene glycol) monoethyl ether methacrylate] (P(TEGMA-EE) brushes on silicon wafers. They obtained layers up to 20 nm on which they could cultivate human fibroblast cells and harvest confluent cell sheets by lowering the temperature to 17.5°C (Dworak et al., ACS Appl. Mater. Interfaces, 2013, 5, 2197).

Despite the precise control over coating thickness and the well-defined polymer architecture, the main drawback of CLRPs is that they cannot easily be transferred to plastic cell culture substrates without a costly pre-functionalization.

In grafting-to approaches, preformed polymer chains are immobilized and/or crosslinked onto cell culture substrates. Many different coating techniques, like for example spin coating, spray coating and dip coating, are available to deposit the polymeric material onto a surface. Spin coating is a convenient method of film deposition and allows for a simple control of the deposited film thickness by adjusting the concentration of the depositing solution. However, spin coating is a sensitive and costly coating method requiring specific equipment and is not suitable for every type of substrate geometry.

It has been shown that a facile control of polymer layer thickness using the spin coating technique is possible. In particular, a photo-crosslinkable copolymer of pNIPAm with a benzophenone derivative (acrylamidebenzophenone, AcBzPh) as photo-reactive comonomer was spin coated onto polystyrene substrates providing polymer layers with a thickness between 13 and 55 nm. The copolymer was prepared by free radical polymerisation using AIBN as initiator (Nash et al., Soft Matter, 2012, 8, 3889). The authors showed that cells were only growing on polymer films with thicknesses below 30 nm, which is in agreement with the findings of Akiyama et al., who used EBP to functionalize polystyrene substrates with pNIPAm. Further, thermoresponsive polymer films using the same copolymer were prepared on tissue culture plastic surfaces using physical adsorption prior to crosslinking via UV exposure (Healy et al., Macromolecular Bioscience, 2016, DOI 10.1002/mabi. 201600175). The copolymer (0.988 molar ratio NIPAM, 0.012 molar ratio of AcBzPh) was adsorbed above and below the polymers cloud point temperature. Polymer adsorption at a temperature below the cloud point temperature provided polymer films with a thickness of about 13 nm with good cell adhesion and proliferation properties.

Buller et al. used a similar approach to coat silicon wafers with p(MEO₂MA-co-OEGMA-co-BPEM) copolymers which also contained a photo-reactive BP moiety. However, there were no further reports on the performance of their thermoresponsive coatings in the culturing of cells, suggesting a poor cell attachment and proliferation (Buller et al., Soft Matter, 2013, 9, 929).

The group of Werner et al used the plasma- and electron beam method to immobilize their thermoresponsive polymers onto different substrates after spin coating. For example, they used low pressure argon plasma to graft pNIPAm and poly(NIPAm-co-DEGMA) copolymer chains onto fluorocarbon substrates. They were able to show the detachment of confluent L292 fibroblast and human corneal endothelial (HCEC) cell-sheets and reported faster detachment times for the copolymer compared to the pure pNIPAm coatings (Nitschke et al., Journal of Biomedical Materials Research Part A, 4, 1003).

Werner et al. also immobilized poly(vinyl methyl ether) (PVME) and its according copolymer with maleic anhydride (PVME-MA) onto polystyrene substrates using a low energy electron beam. They obtained films with thicknesses up to 100 µm on which they could cultivate HCEC (Gramm et al., Polymer Letters, 2011, 5(11), 970). In a further approach, they coated hydrophilized polystyrene coated cover slips with a spin coated polymer blend layer consisting of PVME (50%), pNIPAm (40%) and PVMEMA (10%). They were able to culture and harvest transplantable HCEC cell-sheets with maintained morphology, viability and functionality (Teichmann et al., Science and Technology for Advanced Materials, 2015, 16, 045003).

Another example for a grafting-to approach was published by Dworak et al., who immobilized thermoresponsive poly(2-isopropyl-2-oxazoline) (PIPOx) and Poly[(2-ethyl-2-oxazoline)-co-(2-nonyl-2-oxazoline)] (PENOx) onto functionalized silicon surfaces by quenching the living cationic polymerization with amino groups presented on the surface. They obtained coating thicknesses between 4 and 11 nm and where able to adjust the switching temperature of the layers via the monomer ratio as well as the polymer molecular weight. They cultivated and harvested dermal fibroblast cell-sheets and were able to show that the surfaces did not impact the gene expression of the cells (Dworak et al., J. Mater. Sci. Mater. Med., 2014, 25, 1149). However, it is worth noting that similarly to the CLRP methods, such systems are not easily transferable to plastic cell culture substrates.

An objective of the invention is to provide a thermoresponsive polymer for a cell sheet engineering approach that can be carried out in an easy manageable manner.

This object is solved by a thermoresponsive polymer according to claim 1, a method for obtaining the same according to claim 7 and a cell substrate comprising the thermoresponsive polymer according to claim 11.

Accordingly, a thermoresponsive polymer is provided comprising
a linear polyether chain of the general formula (I)

   Nu-[CH₂-CR¹R²O]ₘ-[CH₂-CR³R⁴O]ₙ-[CH₂-CR⁵R⁶O]ₒ-[CH₂-CR⁷R⁸O]ₚ-H

   wherein
   - R¹, R³, R⁵ and R⁷ are in each case H or a C₁-C₁₀ alkyl side chain, wherein R¹, R³, R⁵ and R⁷ may be the same or different,
   - R² and R⁴ are in each case a side chain comprising at least one thermoresponsive moiety, wherein R² and R⁴ may be the same or different;
   - R⁶ and R⁸ are in each case H, a moiety containing at least one C=C double bond or a photo-crosslinkable moiety selected from a group of aromatic and heteroaromatic compounds, wherein R⁶ and R⁸ may the same or different, wherein at least one of R⁶ and R⁸ has to be a photo-crosslinkable moiety,
   - Nu is nucleophilic moiety selected from a group comprising halogen, azide or alkoxy groups.
   - m, n are in each case or in sum 10-5000, preferably 10-1000, more preferably 100-500; and
   - o, p are in each case 1-20 or in sum 2-20, preferably 3-15, more preferably 5-10.

A thermoresponsive polymer is provided, wherein the photo-crosslinkable moiety is arranged predominantly in one of the end regions of the polymer chain, i.e. the photo-crosslinkable moiety is present as a block at one end of the polymer chain. One or two more hydrophilic domains comprising the thermoresponsive moieties are linked to a more hydrophobic end domain of the polymer chain. Thus, there is no statistical distribution of the photo-crosslinkable moiety within the polymer chain.

The block arrangement of the photo-crosslinkable moiety at one end region of the polymer chain can be obtained by a sequential polymerisation, in particular a sequential ionic polymerisation, as will be described later in detail below. The sequential polymerisation allows in a first step the synthesis of the thermoresponsiveregion of the polymer with the thermoresponsive moieties and in the second step the hydrophobic photo-crosslinkable aromatic moiety is linked to the polymer chain.

The present thermoresponsive polymers may provide an arrangement of the thermoresponsive polymer on a substrate in the configuration of a pancake, mushroom or brushes wherein the photo-crosslinkable moieties (as heads) are bound to the substrate and the polymer chain (as tails) are directed away from the substrate.

In an embodiment of the present polymer moieties R¹,R³, R⁵ and R⁷ are H or a C1-C6 alkyl chain, in particular a methyl or ethyl group. H is the most preferred moiety.

In another embodiment of the present polymer the moieties R² and R⁴ are in each case an alkyl group functionalized with a OH or a C1-C12 alkyl or alkyl ether group.

In a preferred embodiment, the moieties R² and R⁴ are a C1-C6 alkyl group functionalized with a C1-C6 alkoxy, in particular methoxy and ethoxy. For example, moieties R² and R⁴ may be a methyl or ethyl group that is further functionalized with an alkoxy group, such as methoxy or ethoxy group as hydrophilic moieties. In the most preferred case R² is -CH₂-OCH₃ and R⁴ is - CH₂-OC₂H₅. Further possible variants of R² and R⁴ will be apparent further below.

In a further variant of the present polymer moieties R⁶ and R⁸ are a C2-C10 alkyl or alkyl ether with at least one double or triple bond, preferably at least one terminal double bond. R⁶ and R⁸ may be -CH₂OCH₂CHCH₂, -CH₂OCHCH₂, , -CH₂OCH₂CH₂OCHCH₂, -CH₂OC(O)CHCH₂, CH₂OC(O)C(CH₃)CH₂, -CH₂OCH₂CCH, -CH₂OCH₂CH₂CH₂SCH₂CH₂NH₂ or - CH₂OCH₂CH₂CH₂SCH₂CH₂COOH.

In a preferred embodiment of the polymer in case R⁶ and/or R⁸ are a photo-crosslinkable moiety, said moiety comprises a C6-C20 aryl as a photo initiator structure. Such structures may be an aromatic ketone, such as benzophenone, anthraquinone, xanthone and thioxanthone. The photo initiator structures may be further functionalized as depicted below: 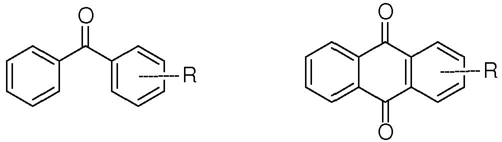 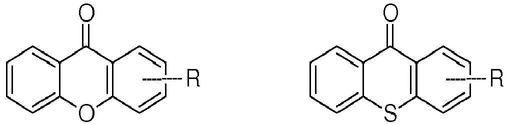 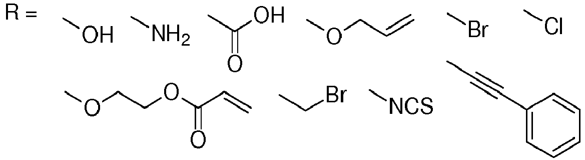 The photo-crosslinkable moiety may be linked to the polymer chain or backbone via an ether group or an amide group. In general, any suitable linkage is possible and depends mainly on the functionalities of the side chains used in the thermoresponsive polymers. For example, in case an alkoxy moiety is used as thermoresponsive functionality then the photo-cross-linkable moiety R6 is preferably linked via an ether bridge to the polymer chain.

The nucleophilic moiety Nu may be a halogen, in particular Cl or Br, an azide group or an alkoxy group that may be further substituted with protected functional groups and/or have a branched structure. Possible Nu moieties may be linear or branched glycerol structures. Preferred Nu moieties are -OCH₃, -O(CH₂)ₙX" -(OCH₂)₄CH₃, -(OCH₂)₃CCH₂OCH₂C(CH₂O)-, where X is selected from a number of protected functional groups (e.g. a benzyl protected thiol).

In a preferred embodiment the present polymer is of the general formula II

Nu-[CH₂-CHR²O]ₘ-[CH₂-CHR⁴O]ₙ-[CH₂-CHR⁶O]ₒ-[CH₂-CHR⁸O]ₚ-H

wherein R², R⁴, R⁶, R⁸, Nu, m, n, o and p have the above meaning.

In another embodiment the present polymer is of the general formula III

Nu-[CH₂-CHCH₂OR^{2'}-O]ₘ-[CH₂-CHCH₂OR^{4'}-O]ₙ-[CH₂-CHR⁶-O]ₒ-[CH₂-CHR⁸O]ₚ-H

wherein
- R^{2'} and R^{4'} are H or C1-C6 alkyl that may be interrupted by one or multiple oxygen atoms, sulphur atoms, substituted nitrogen atoms, -CO-, -C(O)O-, -OC(O)-, - OC(O)O-, -NHC(O)O-, -OC(O)NH- and/or a double bond; and
- R⁶, R⁸, Nu, m, n, o and p have the above meaning.

R^{2'} and R^{4'} are in particular - CH₃, -CH₂CH₃, -CH(CH₃)₂, -C(CH₃)₃, -C(O)CH₃, - CH(CH₃)OCH₂CH₃, -CH₂CH₂OCH₃, -CH₂CH₂OCH₂CH₃, -CH₂CH(ORₓ,_{y})CH₂ORₓ,_{y}, wherein R_{x,y}, are a C1-C10 alkyl side chain that may be further functionalized with a hydrophilic group such as OH, alkoxy and others.

In yet another more preferred embodiment the present polymer is of the general formulae IV

Nu-[CH₂-CHCH₂OCH₃-O]ₘ-[CH₂-CHCH₂OC₂H₅-O]ₙ-[CH₂-CHR⁶-O]ₒ-H

wherein R⁶, Nu, m, n and o have the above meaning.

In yet further embodiments the present thermoresponsive polymer may be of the formula V

Nu-[CH₂-CHCH₂OCH₃-O]ₘ-[CH₂-CHCH₂OC₂H₅-O]ₙ-[CH₂-CHCH₂OC₂H₄R⁶-O]ₒ-H

wherein R⁶, Nu, m, n and o have the above meaning.
and/or of the formulae VI

Br-[CH₂-CHCH₂OCH₃-O]ₘ-[CH₂-CHCH₂OC₂H₅-O]ₙ-[CH₂-CHCH₂OC₂H₄OC₁₃H₉O-O]ₒ-H,

wherein m, n and o have the above meaning.

The cloud point temperature of the present thermoresponsive copolymer is preferably in a range between 0 °C and 40 °C. More preferably the cloud point temperature of the present thermoresponsive copolymer is within the physiologically relevant range between 10 and 37°C.

The cloud point temperature of the thermoresponsive copolymer depends strongly on the side chains or different moieties. Thus, for example in case R² and R⁴ are ethoxy groups, the cloud point temperature of the polymer would be 15°C or lower, depending on the polymer chain length and concentration in aqueous solution or the polymer chain density on the functionalized culture substrate.

As mentioned previously the present thermoresponsive polymer is obtained in a sequential polymerisation process, in particular a sequential ionic polymerisation process.

Such a process or method for synthesizing a present thermoresponsive polymer may comprise the following steps:
- providing a first monomer comprising moieties R¹ and R²;
- optionally providing a second monomer comprising moiety R³ and R⁴;
- reacting the monomers in the presence of at least one activator and at least one initiator comprising the Nu moiety;
- adding at least compound comprising moieties R⁶ and R⁸ to the polymer mixture; and
- working up the obtained polymer mixture for retrieving the thermoresponsive polymer.

In the present method the first monomer comprising moieties R¹ and R² and the second monomer comprising moieties R³ and R⁴ are in each case epoxides comprising the respective moieties R¹ and R², R³ and R⁴.

Similarly, it is preferred that at least one compound comprising moiety R⁶ and R⁸ is an epoxide functionalized with the respective moiety R⁶ and R⁸.

In an embodiment of the present method the activator may be at least one Lewis acid, in particular an Aluminium containing compound, in particular trialkylaluminum, such as Triisobutylaluminum (i-Bu₃Al).

In another embodiment of the present method at least one initiator is an alkali metal containing compound or an ammonium halide, in particular an alkylated ammonium bromide such as NOct₄Br. The use of an ammonium halide as initiator is however preferred since by replacing alkali metals as counter ions of the growing chain end by bulkier ammonium counterions NR₄⁺ increases the polymerisation rate constant about 5 to 50 times depending on the respective counter ion.

The monomer activation requires an excess of the Lewis acid, such as an aluminium compound, with respect to the ammonium compound as activator. The ratio is preferably aluminium compound / ammonium compound >1, for example [i-Bu₃Al]/[NOct₄Br] > 1.

The sequential polymerisation is preferably carried out at low temperatures. For example, the polymerisation may be carried out at temperatures between 0 °C and 25 °C (or room temperature).

In the most preferred embodiment of the present method the epoxide monomers glycidyl methyl ether (GME) and ethyl glycidyl ether (EGE) are copolymerized in the first step, preferably in a ratio of GME/EGE = 1:3, at a low temperature, preferably at 0°C The copolymerization provides a thermoresponsive copolymer with a statistical distribution of GME and EGE units within the polymer chain (r_{GME} = 0.98; r_{EGE} = 0.95).

In the second step, a photo-reactive crosslinkable compound is added to the polymer mixture.

For example, in a first synthesis strategy an epoxide functionalized benzophenone is added and undergoes polymerization at the terminus of the polymer chain. Thus, there is no reaction with the side chains (e.g. methoxy or ethoxy functionalized side chains) of the further growing polymer chain. Since the photo-reactive compound is added only after a polymer chain has already formed, the photo-reactive compound is only incorporated into the end region of the polymer chain and thus forms a type of anchoring block that is useful for adhesion and linkage to a suitable polymer substrate.

The polymerisation reaction according to the first synthesis strategy is exemplarily shown in the following reaction scheme:

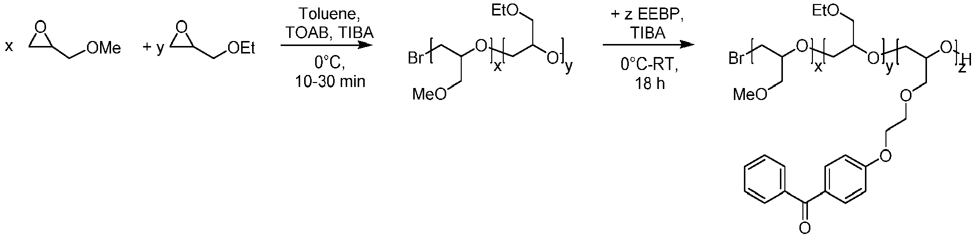

The thermoresponsive polymer obtained according to the above reaction schemes is a poly(GME-stat-EGE-block-EEBP) with a molecular weight of between 25.000 and 30.000 g/mol, preferably about 28.000 g/mol with a GME/EGE ratio of 1:3 and 3.3 EEBP units per chain (3.3 units ≈1.000 g/mol).

In a second synthesis strategy, an epoxide monomer such as allyl glycidyl ether (AGE) is added in the second step of the polymerization. The allyl side chains of this block-copolymer can be functionalized with the photoinitiator after polymerization in order to obtain the photo-crosslinkable anchor block.

A reaction route according to the second synthesis strategy is exemplarily shown in the following reaction scheme:

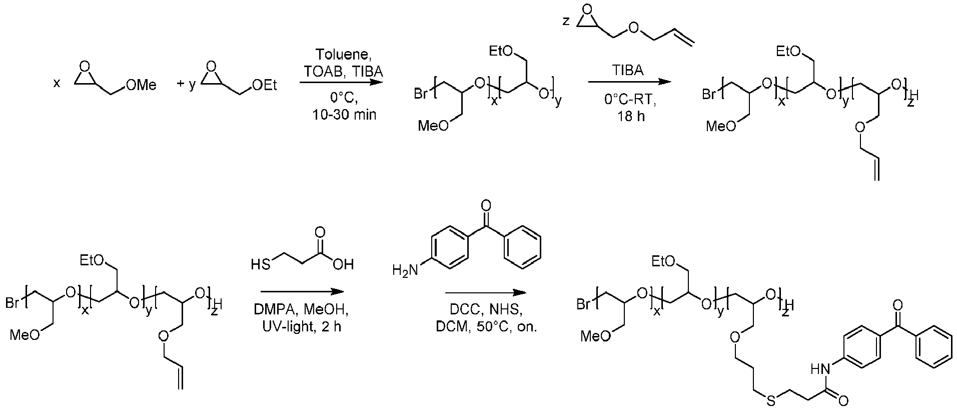

The GME/EGE ratio can be adjusted depending on the desired cloud point temperature between 1:1 and 100% EGE. A high percentage of EGE (>85%) may allow for a better cell adhesion and cell growth.

As mentioned above the present thermoresponsive polymer is suitable for coating a substrate for cell culturing.

Accordingly, a cell culture support comprising a substrate and a coating layer comprising at least one of the above described thermoresponsive polymers is provided.

In an embodiment of the present cell culture support, the substrate is selected from the group consisting of aromatic and non-aromatic polymeric materials comprising aliphatic groups or functional groups like hydroxyl or amine groups. This allows for a covalent anchoring or covalent immobilization of the thermoresponsive polymer (caused by C-H-insertion of the photo-crosslinkable moiety of the polymer) to the substrate by exposure to irradiation.

In a preferred variant, the substrate is based on a polymer selected from a group comprising polystyrene (PS), polyethylene terephthalate (PET), polycarbonate (PC), polyvinyl chloride (PVC), polypropylene (PP), polyurethane (PU), cyclic olefin copolymer (COC) and polyethylene (PE).

Furthermore, the polymeric materials used as substrate may be of any shape. Thus, the coating layer may be formed on any shaped substrate independent on the substrate's geometry. The substrate can be provided in any useful form including, but not limited to, dishes, multiwell-plates, tissue culture flasks, thin films, sheets, membranes, filters, nonwoven or woven fibers, hollow or solid beads, bottles, plates, tubes, rods, pipes, or wafers.

The thermoresponsive polymer is coated onto the polymeric substrate by adsorption (for example by incubating the polymeric substrate in a polymer solution for a certain period of time like 30 min). The obtained polymer coating is stable. Thus, no spin coating or any other elaborate coating technique is required, which allows the functionalization of any surface-structure and -geometry.

In a particular preferred embodiment, the thermoresponsive polymer may be disposed and bonded to the substrate in form of a monolayer. The mono- or multilayer may have a thickness between 0.5 and 1 µm, preferably between 0.5 and 500 nm, more preferably between 0.5 and 40 nm. It is for example possible to obtain monolayer with a thickness as low as 0.5 to 2 nm. The thickness of the monolayer is mostly dependent on the length of the thermoresponsive part of the polymer.

The polymeric substrate coated with the thermoresponsive polymer is subsequently irradiated for example with UV light (365 nm). This causes a covalent bonding of the thermoresponsive polymer to the surface of the polymeric substrate. The bonding is effected by a C-H-insertion of the photo-crosslinkable anchor moiety (such as benzophenone).

The present thermoresponsive polymer system has multiple advantages over known systems:
- substrate coating can be achieved using a rather dilute polymer solution, i.e. the amount of polymer can be kept at a minimum;
- defined monolayer thickness (via polymer length of thermoresponsive part of the polymer);
- stability of the adsorbed layers against washing with ethanol, water, phosphate buffered saline (PBS) or cell culture medium under high shear flow;
- better flexibility (i.e. faster dehydration and hydration at the phase transition temperature) due to no crosslinking in the thermoresponsive polymer tails.
- use of a photo-reactive moiety which enables both the adsorption to the substrate and the subsequent immobilization via UV-irradiation

After removal of excess non-bound polymers from the substrate surface the polymer coated substrates can be used for cell cultivation.

Accordingly, a method for making a cell sheet is provided comprising the steps of:
- providing at least one substrate;
- providing at least one thermoresponsive polymer;
- adsorbing the at least one thermoresponsive polymer on the at least one substrate;
- irradiating the thermoresponsive polymer adsorbed to at least one substrate, preferably by using UV light, to provide a cell culture support;
- depositing a medium comprising cells onto the cell culture support;
- growing cell layers at temperatures above the cloud point temperature of the thermoresponsive polymer;
- detaching the cultured cell layers by lowering the temperature of the cell culture below the cloud point temperature; and
- harvesting the cultured cell layers to provide a cell sheet.

In an embodiment of the present cell cultivation method the cells used for growing cell sheets are selected from a group consisting of, but not necessarily limited to, fibroblasts, myoblasts, myotube cells, corneal cells, vascular endothelial cells, smooth muscle cells, cardiomyocytes, dermal cells, epidermal cells, mucosal epithelial cells, mesenchymal stem cells, ES cells, iPS cells, osteoblasts, osteocytes, chondrocytes, fat cells, neurons, hair root cells, dental pulp stem cells, ß-cells, hepatocytes, and combinations thereof.

The cells bond to the polymer are cultured at a physiological range between 32 and 37°C. For detaching the cultured cell layer the temperature is lowered to a value below the culturing temperature, preferably to room temperature between 20 and 25°C. It is, however, also possible to lower the temperature to 10°C and below, even to 0°C. This could be useful for cells with a strong adhesion to the cell culture substrate.

The invention is explained in more detail with reference to exemplary embodiments and figures. It shows:

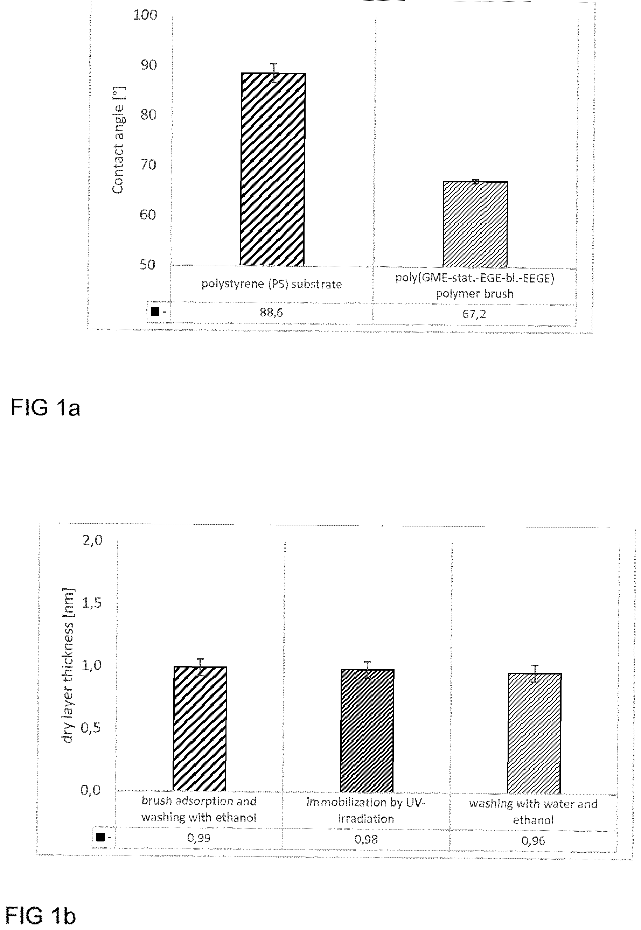

- Figure 1 a: a diagram illustrating the water contact angle of a non-coated substrate and a substrate coated with a polymer according to the invention;
- Figure 1 b: a diagram illustrating the layer thickness of a polymer layer coated on a substrate;
- Figure 2: cell proliferation on non-coated PS substrates, a PS substrate coated with a polymer according to the invention and a TCPS substrate as control.

### Experimental Part

### Materials

All chemicals and solvents were purchased from Sigma-Aldrich (Steinheim, Germany) and used without further purification unless stated otherwise. GME, EGE and 4-hydroxybenzophenone (98%) were purchased from TCI GmbH (Eschborn, Germany). The monomers GME and EGE were dried and distilled over CaH₂ and stored over 3 A molecular sieve purchased from Carl Roth GmbH + Co. KG (Karlsruhe, Germany). Triisobutylaluminum (1.1 M in toluene), epichlorohydrin (99%), 2-bromoethanol (98%) and benzophenone (99%) were purchased from Acros Organics (Geel, Belgium). Diethyl ether (Et₂O) was supplied by VWR Chemicals (Fontenay-sous-Bois, France or Leuven, Belgium) and was distilled before use in order to remove the stabilizer. Sodium sulfate (99%), sodium hydroxide (99%) and potassium carbonate (99%) were supplied by Carl Roth GmbH + Co. KG (Karlsruhe, Germany). Toluene predried via the solvent system MB SPS-800 from M. Braun GmbH (Garching, Germany) was refluxed with elemental sodium and a pinch of benzophenone and distilled on 3 A molecular sieve prior to use. Pre-wetted regenerated cellulose dialysis tubes (molecular weight cut-off (MWCO) 3500 g mol⁻¹, Spectra/Por^{®} 6 Dialysis membrane) from Spectrum Labs were purchased from Carl Roth GmbH + Co. KG.

### Analytical Methods

¹H and ¹³C NMR spectra were recorded on a Bruker Advance 3 operating at 700 MHz or on Joel ECX at 400 MHz and 500 MHz, respectively, measured at concentrations of 100 mg mL-¹ in CDCl₃. Chemical shifts were reported in δ (ppm) and referenced to the respective solvent. Gel permeation chromatography (GPC) was conducted on an Agilent 1100 Series instrument in THF as the eluent solvent with concentrations of 3.5 mg mL⁻¹ and a flow rate of 1 mL min⁻¹ at 25 °C. Three columns Suprema Lux 100, Suprema 1000 and Suprema Lux 3000 with the dimensions: 8 x 300 nm, particle size: 10 µm from PSS (Mainz, Germany) were used in-line with a refractive index detector. Calibration was performed with polystyrene standards. IR measurements were conducted on a Nicolet Avatar 32 FT-IR with Smart iTR accessory. Dynamic light scattering (DLS) was performed on a Malvern Zetasizer Nano-ZS analyzer (Malvern Instruments) equipped with a 4 mW He-Ne laser (λ = 633 nm), at concentrations of 10 mg mL⁻¹ in ethanol at 20 °C. Measurements were performed in PS-latex cuvettes with a refractive index (RI) of 1.590 and low absorption of 0.01 and each probe was equilibrated for 120 s before measuring.

### Synthesis of 4-(2-Hydroxyethoxy)benzophenone (HEBP)

4-Hydroxybenzophenone (10.0 g, 50.45 mmol) was dissolved in 110 ml acetone, to which 14.0 g K₂CO₃ (101.3) was added. The mixture was stirred under reflux for 30 min. 2-Bromoethanol (25.32 g, 202.6 mmol) was added dropwise with a syringe during a course of 30 min. The mixture was left stirring under reflux for 18 h. The solvent was evaporated and the product was taken up in diethyl ether. After washing with water 3 times, the organic phase was separated, dried over sodium sulfate and the solvent was evaporated. The crude product was obtained as a crystalline white solid with a yield of 87 %. ¹H NMR (400 MHz, CDCl₃): δ(ppm) = 7.82, 7.80, 7.75, 7.73, 7.56., 7.46, 6.97, 6.95 (9H, benzophenone), 4.15 (t, -OCH₂CH₂OH), 3.99 (t, - O-CH₂CH₂OH), 2.44 (s, -OCH₂CH₂OH); ¹³C NMR (101 MHz, CDCl₃): δ(ppm) = 195.77 (C=O), 162.41 (C-O), 138.21, 132.69, 132.10, 130.49, 129.83, 128.30, 114.18 (9C, benzophenone), 69.53 (-OCH₂CH₂OH), 61.30 (-O-CH₂CH₂OH).

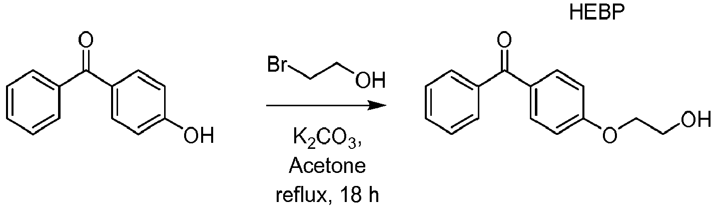

### Synthesis of 4-[2-(2,3-epoxypropoxy)ethoxy]benzophenone (EEBP)

EEBP was synthesized according to a procedure reported by Jabeen et al.² A solution of HEBP (7.8 g, 32.2 mmol) in epichlorohydrine (50 ml, 644.4 mmol) was treated with sodium hydroxide (3.2 g, 80.5 mmol). The suspension was refluxed for 5 h and stirred at room temperature overnight. The residue was filtered of and washed with diethyl ether. The solvent was evaporated and the remaining opalescent oil was taken up in dichloromethane. The organic solution was washed with water several times and dried over anhydrous sodium sulfate. The product was purified by column chromatography using a 1:1 mixture of ethyl acetate and hexane. After evaporating the solvent, a white solid (yield: 7.1 g, 80 %) was obtained. ¹H NMR (400 MHz, CDCl₃): δ(ppm) = 7.82, 7.80, 7.75, 7.73, 7.57, 7.46, 6.99, 6.97 (9H, benzophenone), 4.21 (t, -OCH₂CH₂O-), 3.92 (t, -OCH₂CH₂O-), 3.88, 3.49 (dd, -O-CH₂-epoxide) 3.19 (1 H, m, CH - *epoxide)* 2.81 (1H, dd, CH₂- *epoxide),* 2.63 (1H, dd, CH₂- *epoxide*)*;* ¹³C NMR (101 MHz, CDCl₃): δ(ppm) = 195.63 (C=O), 162.47 (C-O), 138.33, 132.62, 132.01, 130.43, 129.82, 128.29, 114.23 (9C, benzophenone), 72.24 (-OCH₂CH₂O-), 69.73 (-O-CH₂-epoxide) 67.69 (-OCH₂CH₂O-), 50.92 (CH - *epoxide),* 44.21 (CH₂ - *epoxide).*

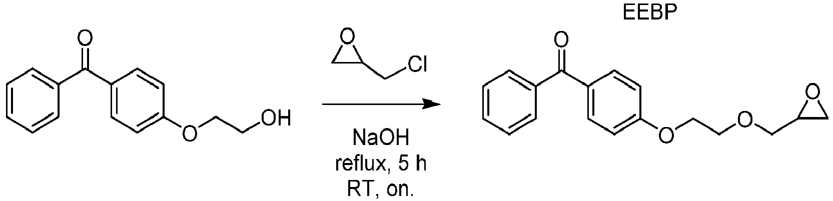

### Synthesis of poly(GME-stat.-EGE-block-EEBP) terpolymers

The terpolymer was synthesized by anionic, monomer activated ring-opening copolymerization according to literature with slight modifications using tetraoctylammonium bromide (NOct₄B) as initiator and triisobutylaluminum (*i*-Bu3Al) as activator.3-5 NOct4Br (61.2 mg, 112 mmol) was dried at 110 °C in HV and dissolved in freshly distilled, dry toluene (15 ml). The monomers GME (0.5 g, 5.7 mmol) and EGE (1.74 g, 17.0 mmol) were added to the solution and cooled in an ice bath. By the addition of *i*-Bu₃Al (1.1 M in toluene) (0.41 ml, 447.7 mmol) at 0 °C the polymerization was started. After 10 min at 0 °C EEBP (0.1 M in toluene) (7.5 ml, 0.75 mmol) was added and the reaction temperature was allowed to raise to room temperature, while the reaction mixture was stirred for minimum an additional 45 min. After quenching with 2 mL of water, two spoons of sodium sulfate were added in order to capture the hydrolyzed aluminum compound together with traces of water. After filtration of the solid and concentration of the filtrate under reduced pressure, the raw product was dissolved in Et₂O in order to precipitate residual aluminum hydroxide and the majority of the tetraoctylammonium salts. In the cold, the mixture was centrifuged and the supernatant was decanted, those two steps were repeated, after storing of the supernatant in the fridge overnight to provoke further precipitation, followed by concentration. For further purification the product was dialyzed in methanol in order to remove traces of impurities. Afterwards the pure polymer solution was concentrated under reduced pressure and dried in HV to yield a clear and viscous oil. Yield: 2.32 g (93.7%) ¹H-NMR (400 MHz; CDCl₃): δ(ppm) = 7.80-6.95 (m, benzophenone) 3.59-3.38 (m, polymer backbone + CH₃CH₂O + OCH₂CH₂O); 3.30 (s, OCH₃); 1.13 (t, CH₃CH₂O). ¹³C-NMR (101 MHz; CDCl₃): δ(ppm) = 195.63 (C=O), 162.47, 138.33, 132.62, 132.01, 130.43, 129.82, 128.29, 114.23 (benzophenone), 78.9-78.5, 72.8, 70.6-69.8, 66.7 (polymer backbone + CH₃CH₂O + OCH₂CH₂O); 59.2 (OCH₃); 15.3 (CH₃CH₂O). GPC: Mₙ = 26797 g mol⁻¹, PDI = 1.05.

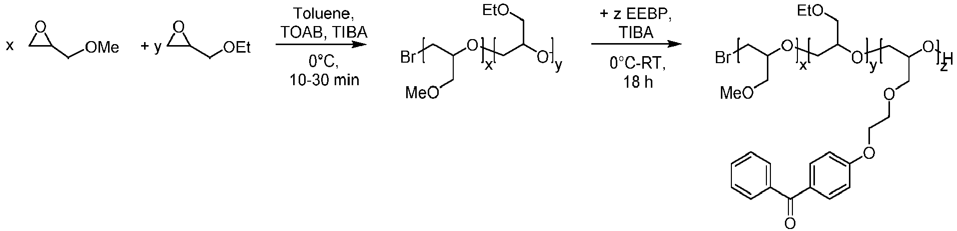

### Spin coating

Silicon wafers were cut into quadratic samples (11 x 11 mm), cleaned by rinsing with ethanol and dried under a stream of nitrogen. The samples were spin coated for 60 s at 3000 rpm using a solution of PS in toluene with a concentration of 0.5 wt.-% (30 µl) and dried under ambient conditions.

### Surface preparation

Polystyrene Petry dishes (35 x 10 mm) and polystyrene coated silicon wafers (11 x 11 mm) were incubated in a 0.01 mM poly(GME-stat.-EGE-block-EEBP) solution in ethanol for 30 min. After the adsorption process, the solution was removed and the surfaces incubated with ethanol and water multiple times and thoroughly rinsed afterwards. After drying under a stream of nitrogen the samples were irradiated with UV-light (4 W/cm²) using an LED with a wavelength of 365 nm. The PS Petry dishes were used for cell culture experiments and the functionalized silicon wafers were characterized by water contact angle measurements and ellipsometry.

### Contact angle measurements

The sessile drop method was applied to determine the static water contact angle before and after polymer adsorption on polystyrene coated silicon surfaces. Therefore, a drop of MilliQ water (2 µL) was placed onto the respective surface and a photo was taken. Contact angles were determined with an ellipse fitting model. For each substrate, contact angles were measured on three different spots to determine the homogeneity of the coating and at least four independent substrates (n=4) were investigated to determine reproducibility. Mean contact angles of each substrate were averaged.

The diagram in Figure 1 a shows the contact angles of drops of water (2 µl) on bare polystyrene (PS) coated silicon wafers and thermoresponsive polymer coatings after adsorption, UV-immobilization and washing with water and ethanol.

### Ellipsometry

The dry layer thickness of the polymer coatings was determined by multi-angle spectroscopic ellipsometry at 70 °. The thicknesses of the silicon dioxide layer and of the spin coated polystyrene layer were determined separately before and after spin coating, respectively, and average values of at least three different spots on the surface were taken as fixed values for the modeling of the adsorbed polymer layer. The layer thickness was measured at wavelengths from 370 nm to 1050 nm and was fitted using a model consisting of the previously measured SiO₂ and PS layers with fixed parameters, a Cauchy layer with fixed refractive index of n = 1.5 and air as the surrounding medium.

The diagram in Figure 1b shows the dry layer thickness determined by spectroscopic ellipsometry of the thermoresponsive polymer coatings on polystyrene (PS) coated silicon wafers after adsorption, UV-immobilization and washing with water and ethanol. Even after multiple washing and extraction the polymer forms a stable coating layer.

### Cell adhesion and detachment

Cell culture studies with primary human fibroblasts were performed on non-treated coated and uncoated polystyrene Petry dishes (Dow Corning Inc., 35 mm x 10 mm). Conventional tissue culture polystyrene (TCPS) Petry dishes (VWR^{®}, 35 mm x 10 mm) were used as control. Primary fibroblasts were cultured in DMEM (life technologies™, Paisley, UK) supplemented with 10% FBS, 100 units/ml penicillin, and 100 µg/ml streptomycin. The dishes were washed with 70% ethanol and PBS before seeding. 1.6 x 10⁶ cells in 2 ml DMEM (low glucose, 1 g/l) were seeded in the TCPS, uncoated and coated dishes followed by incubation at 37°C and 5% CO₂ until confluence was reached (24 h). Cells were monitored after 4 h and 24 h via microscopy (Zeiss Observer Z1, Jena, Germany, phase contrast). For temperature triggered detachment, substrates with adherent and confluent cell sheets were incubated with PBS at 20°C and 37°C for 10 and 5 min, respectively, and kept at room temperature (20°C) until the cell sheets detached from the surface.

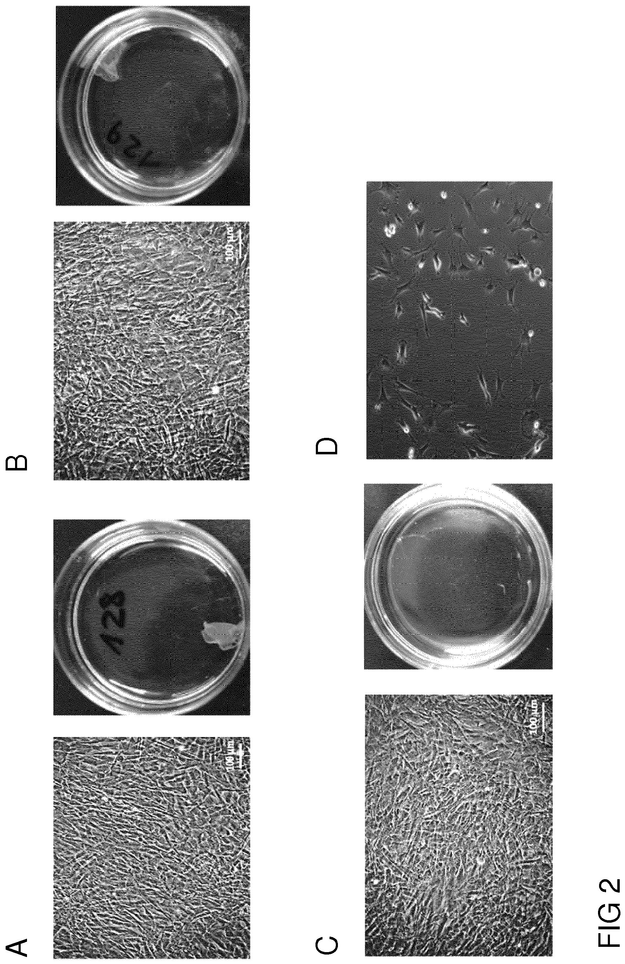

In Figures 2A and 2B proliferation of primary fibroblast cells on thermoresponsive polymer coatings on polystyrene (PS) Petry dishes after 4 h of incubation (left) and detached confluent cell sheets (right) in shown,

In Figure 2C proliferation of primary fibroblast cells on tissue culture polystyrene (TCPS) Petry dish control after 4 h (left) and undetached confluent cell sheet (right) is illustrated. Figure 2D finally shows proliferation of primary fibroblast cells on an untreated polystyrene (PS) Petry dish control. As deducible there is almost no cell growth detectable on the untreated control.

## Claims

1. A thermoresponsive polymer
comprising
a linear polyether chain of the general formulae (I)
Nu-[CH₂-CR¹R²O]ₘ-[CH₂-CR³R⁴O]ₙ-[CH₂-CR⁵R⁶O]ₒ-[CH₂-CR⁷R⁸O]ₚ-H
wherein
- R¹, R³, R⁵ and R⁷ are in each case H or a C₁-C₁₀ alkyl side chain, wherein R¹, R³, R⁵ and R⁷ may be the same or different,
- R² and R⁴ are in each case a side chain comprising at least one thermoresponsive moiety, wherein R² and R⁴ may be the same or different;
- R⁶ and R⁸ are in each case H, a moiety containing at least one C=C double bond or a photo-crosslinkable moiety selected from a group of aromatic and heteroaromatic compounds, wherein R⁶ and R⁸ may the same or different, wherein at least one of R⁶ and R⁸ has to be a photo-crosslinkable moiety,
- Nu is nucleophilic moiety selected from a group comprising halogen, in particular Cl and Br, or azide and alkoxy.
- m, n are in each case or in sum 10-5000, preferably 10-1000, more preferably 100-500;
- o, p are in each case 1-20 or in sum 2-20, preferably 3-15, more preferably 5-10.

2. Polymer according to claim 1, **characterized in that** that R¹, R³, R⁵ and R⁷ are H or a C1-C5 alkyl chain, in particular a methyl or ethyl group.

3. Polymer according to one of the preceding claims, **characterized in that** R² and R⁴ are an alkyl moiety functionalized with a OH or a C1-C12 alkyl or alkyl ether group.

4. Polymer according to one of the preceding claims, **characterized in that** in case R⁶ and/or R⁸ are the photo-crosslinkable moiety said moiety comprises a C6-C20 aryl as a photo initiator structure.

5. Polymer according to one of the preceding claims **characterized in that** it is of the general formula II
Nu-[CH₂-CHR²O]ₘ-[CH₂-CHR⁴O]ₙ-[CH₂-CHR⁶O]ₒ-[CH₂-CHR⁸O]ₚ-H
wherein R², R⁴, R⁶, R⁸, Nu, m, n, o and p have the above meaning.

6. Polymer according to one of the preceding claims **characterized in that** it is of the general formula III
Nu-[CH₂-CHCH₂OR^{2'}-O]ₘ-[CH₂-CHCH₂OR^{4'}-O]ₙ-[CH₂-CHR⁶-O]ₒ-[CH₂-CHR⁸O]ₚ-H
wherein
- R^{2'} and R^{4'} are H or C1-C6 alkyl that may be interrupted by one or multiple oxygen atoms, sulphur atoms, substituted nitrogen atoms, -CO-, -C(O)O-, -OC(O)-, - OC(O)O-, -NHC(O)O-, -OC(O)NH- and/or a double bond; and
- R⁶, R⁸, Nu, m, n, o and p have the above meaning.

7. Method for synthesizing a thermoresponsive polymer according to one of the preceding claims comprising the steps of
- providing a first monomer comprising moieties R¹ and R²;
- optionally providing a second monomer comprising moieties R³ and R⁴;
- reacting the monomers in the presence of at least one activator and at least one initiator comprising the Nu moiety;
- adding at least compound comprising moieties R⁶ and R⁸ to the polymer mixture; and
- working up the obtained polymer mixture for retrieving the thermoresponsive polymer.

8. Method according to claim 7, **characterized in that** the first monomer comprising moieties R¹ and R² and the second monomer comprising moieties R³ and R⁴ are in each case epoxides comprising the respective moieties R¹ and R², R³ and R⁴.

9. Method according to claim 7 or 8, **characterized in that** the at least one compound comprising moiety R⁶ and R⁸ is an epoxide functionalized with the respective moiety R⁶ and R⁸.

10. Method according to one of the claims 7 to 9, **characterized in that** the at least one activator is at least one Lewis acid, in particular an aluminium containing compound, and the at least one initiator is an ammonium halide, in particular an alkylated ammonium bromide.

11. A cell culture support comprising a substrate and a coating layer comprising at least one thermoresponsive polymer according to one of the claims 1 to 6.

12. Support according to claim 11, **characterized in that** the substrate is selected from the group consisting of polymeric materials comprising photo-reactive moieties, in particular alkyl chains.

13. Support according to one of the claims 11 to 12, **characterized in that** substrate is provided in any useful form including dishes, multiwell-plates, tissue culture flasks, thin films, sheets, membranes, filters, nonwoven or woven fibers, hollow or solid beads, bottles, plates, tubes, rods, pipes, or wafers.

14. Method for making a cell sheet comprising the steps of:
- providing at least one substrate;
- providing at least one thermoresponsive polymer according to one of the claims 1 to 6,
- adsorbing the at least one thermoresponsive polymer on the at least one substrate;
- irradiating the thermoresponsive polymer adsorbed to the at least one substrate, preferably by using UV light, to provide a cell culture support; and
- depositing a medium comprising cells onto the cell culture support.
- growing cell layers at temperatures above the cloud point temperature of the thermoresponsive polymer;
- detaching the cultured cell layers by lowering the temperature of the cell culture below the cloud point temperature; and
- harvesting the cultured cell layers to provide a cell sheet.

15. Method according to claim 14, **characterized in that** the cells are selected from a group consisting of, but not necessarily limited to, fibroblasts, myoblasts, myotube cells, corneal cells, vascular endothelial cells, smooth muscle cells, cardiomyocytes, dermal cells, epidermal cells, mucosal epithelial cells, mesenchymal stem cells, ES cells, iPS cells, osteoblasts, osteocytes, chondrocytes, fat cells, neurons, hair root cells, dental pulp stem cells, ß-cells, hepatocytes, and combinations thereof.
